# EUROPEAN PATENT SPECIFICATION

(11) **EP 1 655 378 B1**
(45) Date of publication and mention of the grant of the patent: **05.03.2008**
(21) Application number: 04766886.8
(22) Date of filing: 23.07.2004
(51) Int. Cl.: C12Q 1/68

(54) **METHOD FOR QUANTIFYING AND ASSESSING THE VIABILITY OF NEOSPORA CANINUM IN SAMPLES OF BOVINE SEMEN**
VERFAHREN ZUR QUANTIFIZIERUNG UND BEURTEILUNG DER LEBENSFÄHIGKEIT VON NEOSPORA CANINUM IN PROBEN VON RINDERSPERMA
PROCEDE DE QUANTIFICATION ET D'ESTIMATION DE LA VIABILITE DE NEOSPORA CANINUM DANS DES ECHANTILLONS DE SPERME BOVIN

(30) Priority: 25.07.2003 ES 200301762
(43) Date of publication of application: 10.05.2006
(73) Proprietor: UNIVERSIDAD COMPLUTENSE DE MADRID, 28040 Madrid (ES)
(72) Inventor: ORTEGA MORA, Luis Miguel, Dpto. Sanidad Animal, 28040 Madrid (ES); COLLANTES FERNANDEZ, Esther, E-28040 Madrid (ES); REGIDOR CERRILLO, Javier, E-28040 Madrid (ES); FERRE PEREZ, Ignacio, E-28040 Madrid (ES)
(74) Representative: Carpintero Lopez, Francisco
(86) International application number: PCT/ES2004/000347
(87) International publication number: WO 2005/012563

(56) References cited:
- US-A- 5 942 394
- COLLANTES-FERNANDEZ E. ET AL.: 'Quantitative detection of Neospora caninum in bovine aborted fetuses and experimentally infected mice by real-time PCR' J. CLIN. MICROBIOL. vol. 40, no. 4, 2002, pages 1194 - 1198, XP002996201
- BASZLER T.V. ET AL.: 'Detection by PCR of Neospora caninum in fetal tissues from spontaneous bovine abortions' J. CLIN. MICROBIOL. vol. 37, no. 12, 1999, pages 4059 - 4064, XP002304712
- PEREIRA-BUENO J. ET AL.: 'Evaluation by different diagnostic techniques of bovine abortion associated with Neospora caninum in Spain' VET. PARASITOL. vol. 111, no. 2-3, 2003, pages 143 - 152, XP002996205
- VAN ENGELENBURG F.A. ET AL.: 'Development of a rapid and sensitive polamerase chain reaction assay for detection of bovine herpesvirus type 1 in bovine semen' J. CLIN. MICROBIOL. vol. 31, no. 12, 1993, pages 3129 - 3135, XP002996202
- SANTURDE G. ET AL.: 'Rapid and high sensitivity test for direct detection of bovine herpesvirus-1 genome in clinical samples' VET. MICROBIOL. vol. 49, no. 1-2, 1996, pages 81 - 92, XP002996203
- ORTEGA-MORA L.M. ET AL.: 'Detection of Neospora caninum in semen of bulls' VET. PARASITOL. vol. 117, no. 4, 2003, pages 301 - 308, XP002996204

### SEQUENCE LISTING

<110> Universidad Complutense de Madrid
<120> Procedure for the quantification and evaluation of the viability of *Neospora caninum* in samples of bovine semen.
<140> 200301762
   <141> 2003-07-25
<160> 4
<210> 1
   <211> 20
   <212> DNA
   <213> *Neospora caninum*
<400>
   ACTGGAGGCA CGCTGAACAC 20
<210> 2
   <211> 21
   <212> DNA
   <213> *Neospora caninum*
<400>
   AACAATGCTT CGCAAGAGGA A 21
<210> 3
   <211> 20
   <212> DNA
   <213> *Neospora caninum*
<400>
   TGCCATGGTA ATCCTGCTCA 20
<210> 4
   <211> 21
   <212> DNA
   <213> *Neospora caninum*
<400>
   CCTCAGCCAA GCACATACAC C 21

## Description

### OBJECT OF THE INVENTION

The present invention, as expressed in the statement of this specification, refers to a procedure for the quantification and evaluation of the viability of *N. caninum* in samples of semen. More specifically, the invention refers to the standardisation and application of a quantitative real PCR technique for the quantification of the DNA of the parasite *N. caninum* in samples of fresh or frozen semen and the determination of the viability of the parasite in this fluid using a bioassay in mice of the BALB/c *nu*/*nu* strain. The aforementioned has great relevance, since the application of these techniques will enable the detection of the presence of *N. caninum* in semen and its viability and its infective capacity to be determined. This fact has valuable applications, particularly in the bovine species, where artificial insemination is a normal practice and the use of sensitive and specific diagnostic techniques are needed at the time of determining the health state of the bovine studs, particularly in artificial insemination centres and in extensive exploitations, where natural mounting has great importance.

### BACKGROUND

*Neospora caninum* is a protozoan parasite of the Apicomplexa group described in 1989 as an agent causing abortion and neonatal mortality in cattle (Thilsted & Dubey 1989, J. Vet. Diagnc. Invest. 1: 205-09). The infection has also been described in other hosts such as the goat, sheep, horse, camel, water buffalo, deer, coyote and fox, although the disease has a greater importance in cattle and in the dog (Dubey 1999, Vet. Parasitol. 84. 349-67).

Bovine neosporosis is considered a parasitic disease of cosmopolitan distribution and one of the most common causes of reproductive failure in the different countries where is has been studied (Trees et al., 1999, Int. J. Parasitol. 29: 1195-2000; Anderson et al., 2000, Anim. Reprod. Sci. 60: 417-431), including Spain (González et al., 1999, Vet. Rec. 144: 145-150; Pereira-Bueno et al., 2003 Vet. Parasitol. 111: 143-152). The most important clinical manifestation of the disease is abortion in pregnant females and generally takes place between the third and ninth month of gestation, the most frequent being around 5-6 months. The affected calves that are born alive can show with neuro-muscular problems, the first signs appearing 4-5 days post-partum, although these can be delayed for up to two weeks. However, the birth of clinically healthy, but chronically infected, calves is the most common (Dubey & Lindsay 1996, Vet. Parasitol. 67: 1-59).

As regards the transmission of the disease, the most recent studies indicate the relatively limited importance of postnatal transmission and the persistence, throughout life, of the congenital infection (Davison et al., 1999, Int. J. Parasitol. 29: 1683-1689; Hietala & Thurmond 1999, Int. J. Parasitol. 29: 1669-1676). The elimination of the parasite in semen and its possible venereal transmission or by means of artificial insemination is backed by different epidemiological facts, such as coinciding with the seasonal increase in antibody titre in females at the beginning of the reproductive stage (Hietala & Thurmond 1999, Int. J. Parasitol. 29: 1669-1676; Pereira-Bueno et al., 2000, Int. J. Parasitol. 30: 906-909), the presence of specific antibodies against parasitic infections in studs (Caetano da Silva et al., sent to Vet. Parasitol. for publication), but above all the seminal elimination demonstrated in other taxonomically very close parasites such as *Toxoplasma gondii* (Spence et al., 1978, Vet. Rec. 102: 38-9; Dubey & Sharma 1980, Am. J. Vet. Res. 41: 749-795).

The possibility of the contamination of bovine semen by other infectious or parasitic agents, and their possible transmission the female receptors has become one of the main concerns of the health authorities as well by the productive centre (Hare 1985, p. 117, In: Office International des Épizooties technical series n° 4). The studs as well as their semen, are subjected to strict health programmes which include periodic diagnosis of the most important diseases in accordance with its natural history. In this sense, the standardisation and application of techniques which enable the possible elimination and transmission routes of neosporosis to be studied, as well as the health state of the studs, particularly in artificial insemination centres, is of utmost importance.

In the current scientific literature different PCR techniques have been described for the detection of the DNA of the parasite (Dubey 1999, Vet. Parasitol. 84: 349-367), as well as real time PCR for the quantification of the parasite in samples of infected tissue (Collantes-Fernandez et al., 2002, J. Clin. Microbiol. 40: 1194-1198). However, these techniques are not standardised for the detection of the parasite in fresh or frozen semen. The inhibition phenomena in PCR for the detection of different infectious agents in semen have been described before by several authors (Chistopher-Hennings et al., 1995, J. Clin. Microbiol. 33: 1730-1734; Santurde et al., 1996, Vet. Microbiol. 49: 81-92; Van Engelenburg et al., 1993, J. Clin. Microbiol. 31: 3129-3235).

The detection and isolation of the parasite *Toxoplasma gondii,* phylogenetically related to *N. caninum,* has also been described in the scientific literature, using a bioassay by inoculation method in a mouse model susceptible to toxoplasmosis (Derouin et al., 1987, J. Clin. Microbiol. 25: 1597-1600), a method used to demonstrate its seminal elimination (Spence et al. 1978, Vet. Rec. 102: 38-9; Dubey & Sharma 1980, Am. J. Vet. Res. 41: 749-95). However, up to now, the development of a standardised method to determine the viability and therefore the infective capacity of *N. caninum* in semen using a bioassay with hosts sensitive to neosporosis, such as the gerbil or immunocompromised mice has not been described.

### DESCRIPTION OF THE INVENTION

Procedure for the quantification and evaluation of the viability of *Neospora caninum* in bovine semen samples.

Moore et al (Veterinary Parasitology, 2003, Vol. 114, p. 247-252) pertains to serological evidence of *N. caninum* infections in beef bulls. Dubey et al (The Korean Journal of Parasitology, 2003, vol. 41 (1), p. 1-16) reviews information on biology, diagnosis, epidemiology and control of neosporosis in animals. US-5942394 document pertains to methods of detection of *N. caninum* comprising, the use of nested PCR amplification of internal transcribed spacer 1 region of ribosomal RNA. However, said documents do not mention describe the detection of *N. caninum* in semen samples.

The object of the invention procedure has as its purpose the quantification of *N. caninum* in fresh or frozen samples of bovine semen and the evaluation of its infective capacity with the objective of determining the use of the animal (or its semen) for reproductive purposes.

In this sense, taking the methods for the extraction of DNA from semen described in the scientific literature (Von Beroldingen et al., 1990; p. 209-223. In H. A. Erlich (ed.) Stockton Press, New York; Van Engelenburg et al., 1993, J. Clin. Microbiol. 31: 3129-3235), as a reference, the present invention provides a procedure for the extraction of the DNA of the parasite from samples of semen and its subsequent quantification by techniques based on the PCR technique. The procedure which is advocated, resolves in a completely satisfactory way the problems previously explained and allows the specific quantification of small amounts of parasite DNA in samples originating from fresh as well as frozen bovine semen.

The technique which has been standardised for the quantification of *N. caninum* in semen samples has been a quantitative real time PCR which uses the SYBR Green I system, which has previously shown a high specificity and sensitivity in infected tissues. This technique has also been applied in the quantification of the 28S rRNA gene of the host, with the purpose of being able to compare the parasite load in different samples and correct for the presence of potential inhibitors in the PCR. This technique has the advantage over conventional PCR techniques by enabling the quantification of DNA, greater speed in processing, possibility of reading the samples in plates with 96 wells and not needing subsequent development in agarose gels. Also, the advantage of using a quantitative PCR in real time with the SYBR Green 1 system lies in the fact that it is not necessary to use fluorescent probes, being able to be used for the quantification of the parasite as well as the gene of the host.

The present invention also refers to a standardised procedure for the evaluation and viability of *N. caninum* in semen. The procedure is based on the inoculation of semen samples into athymic mice of the BALBc *nu*/*un* strain, a host sensitive to infection by *N. caninum.* The appearance of symptoms compatible with the infection and the quantification of specific DNA of *N. caninum* in the brain of the inoculated mouse, demonstrates the infective capacity of the analysed samples..

### DESCRIPTION OF THE DRAWINGS

To complement the description and with the purpose of helping to better understand the characteristics of the invention, the present descriptive report is accompanied by, as an integral part of the same, a drawing in which with an illustrative character and not limiting, the following has been represented:

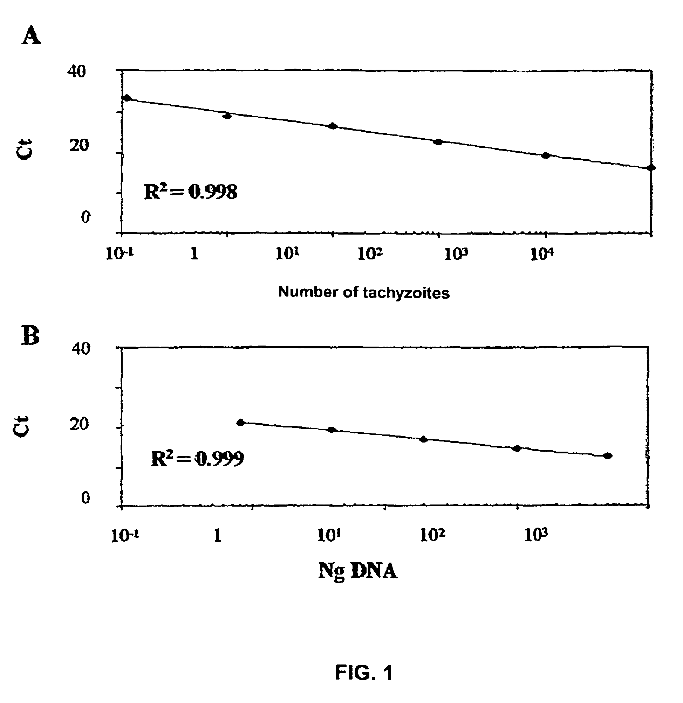

Figure 1. (A) Standard curve for the quantitative determination of *N*. *caninum* constructed with serial decimal dilutions of DNA extracted from cell culture tachyzoites. The Ct values (threshold cycle: cycle in which a sample is considered positive *versus* logarithm of the number of tachyzoites) are represented in the standard curve. (B) Standard curve for the quantification of the host DNA constructed from serial 1:5 dilutions of genome DNA extracted from the host. The Ct values *versus* logarithm of nanograms of DNA are represented.

### MANNER OF CARRYING OUT THE INVENTION

The present invention is illustrated by the following examples:

### Example 1

### Extraction of N. caninum DNA in fresh semen samples:

For the extraction of *N. caninum* DNA in semen samples an extraction method has been standardised using a commercial test: "Genomic-Prep cell and tissue DNA isolation kit" (Amersham Biosciences). With the purpose of determining the sensitivity of the specific quantitative PCR of *N. caninum* in samples of fresh semen, 250 µl aliquots of fresh bovine semen were contaminated with serial decimal dilutions of live tachyzoites previously obtained from cell culture (10⁴-0 tachyzoites/sample). Firstly, the seminal fluid and the cellular fraction were separated from the semen according to the literature (Van Engelenburg et al., 1993, J. Clin. Microbiol. 31: 3129-3235) with the objective of determining the fraction of semen in which the parasite is detected. For this, the samples of semen contaminated with the parasite were centrifuged at 12000 x g for 3 min. The supernatant (seminal fluid: F1) was separated from the cell sediment (cellular fraction: F2) and transferred to a clean tube. Next, an equivalent volume of DNA lysis buffer provided in the commercial test and proteinase K (Sigma) (200 µg/ml) was added to F1 fraction and it is incubated in a Maria bath at 60°C for 1 hour. The sediment originating from the cellular fraction was resuspended in 600 µl of DNA lysis buffer with proteinase K (final concentration 200 µg/ml), being also incubated in a Maria bath at 60°C for 1 h.

At the end of the incubation an RNAse was added (final concentration 20 µg/ml) to the lysate of each fraction and incubated at 37° C for 30 min. Next, for the purification of the DNA, 200 µl of a solution contained in the commercial test was added to precipitate the proteins. The sample was vigorously shaken for 20 s and was centrifuged at 13000-16000 x g for 5 min. The precipitated proteins formed a whitish sediment. The supernatant of the samples was transferred to a clean tube and the sediment with the proteins was discarded.

Finally, the DNA was precipitated by adding 600 µl of cold 100% isopropanol (stored at -20°C), the samples were inverted 50 times and they were incubated at -20°C for 30 min. Then, the samples were centrifuged at 13000-16000 x g/ for 15 min, and the supernatant discarded, carefully, so as not to disturb the precipitated DNA, which appears as a small sediment. The sediment of DNA was washed with 600 µl cold 70% ethanol (-20°C), and the samples were immediately centrifuged at 13000-16000 x g for 5 min. After discarding the ethanol, the samples were left to dry in air until the alcohol was completely evaporated. The sediment was resuspended in 50 µl of the DNA hydration solution contained in the kit and left overnight at room temperature until completely hydrated. Lastly the samples were stored at - 20°C until the PCR was carried out.

### Example 2

### Extraction of the DNA of N. caninum in frozen semen samples

For the extraction of parasite DNA from frozen semen and the determination of the sensitivity of the quantitative PCR in this type of sample, semen was mixed with a commercial diluent (mixture of Tris, glycerol, antibiotics and egg yolk), preparing 250 µl aliquots which were contaminated with serial decimal dilutions of live tachyzoites (10⁴-0 tachyzoites) and were subsequently frozen in liquid nitrogen

With the purpose of eliminating potential inhibitors present in the samples of frozen semen, due to the diluents and taking as a reference the method described by Santurde et al., (1996, Vet. Microbiol. 49: 81-92) the total volume of the sample (250 µl) was passed through a Sephacryl S-400 chromatography column (Amersham Pharmacia). For the assembly of the column, 2 ml of Sephacryl S-400 were added to a polypropylene support (Bio-Rad). The column was placed in a centrifuge tube and was centrifuged at 1000 x g for 2 minutes. The column was equilibrated by adding 2 ml of a 0.3 M solution of sodium acetate at pH 5.3 and was centrifuged again at 1000 x g for 2 min. This procedure was repeated three times. The last column equilibration was performed with a volume of 250 µl of TE pH 7.5 and the column was centrifuged again.
The prepared column was placed in a 1.5 ml tube (without the cap) within a centrifuge tube and the Sephacryl S-400 column was introduced. Next, 250 µl of the frozen semen sample was added over the column and it was centrifuged at 1000 x g for 2 min. The filtrate was collected in the tube, which was processed following the same procedure as for fresh semen, also separating the two fractions: seminal fluid and cellular fraction. The only modification in the DNA extraction procedure lay in the precipitation step with isopropanol, in which 1 µl glycogen of 20 mg/ml added to the 600 µl of isopropanol.

Once was extracted the DNA, the sensitivity of the quantitative PCR was determined in fresh and frozen samples of semen contaminated with serial decimal dilutions of *N. caninum* tachyzoites.

### Example 3

### Quantification of N. caninum in semen samples:

The quantification of *N. caninum* in semen samples was carried out on the DNA extracted from samples of semen according to that described in examples 1 and 2, and a quantitative PCR was employed using the SYBR Green I system (Collantes-Fernández et al., 2002, J. Clin. Microbiol. 40: 1194-1198). The designed oligonucleotides, PF20 (5'-ACTGGAGGCACGCTGAACAC) and PR21 (5'-AACAATGCTTCGCAAGAGGAA) amplify a fragment of 76 pb of the Nc-5 region of the genome of *N. caninum* (Kaufmann et al., 1995, Mol. Cell. Prob. 10: 289-297) between 247 and 323 pb (GenBank accession no. X84238). A similar method was employed for the quantification of the 28S rRNA gene of the host (GenBank accession no. X00525), the designed oligonucleotides for this gene PF28S (5'-TGCCATGGTAATCCTGCTCA) and PR28S (5'-CCTCAGCCAAGCACATACACC) amplify a DNA fragment of 71 pb. The PCR mixture (25µl) consists of 5 µl of DNA, 20 pmoles of each oligonucleotide (Amersham Biosciences), 1 x SYBR Green PCR Buffer, 2 mM MgCl₂, 200 µM of dATP, dCTP, and dGTP, 400 µM dUTP, 0.625 U of Amplitaq Gold DNA polymerase, 0.25 U of AmpErase UNG (uracil-N-glycosylase), all included in the "SYBR Green PCR Core" kit (Applied Biosystems). The amplification consisted of a 2 min cycle at 50°C, 10 min at 95°C, and 40 cycles at 95°C for 15 s and 60°C for 1 min. The reactions or the quantification of *N. caninum* and the 28S rRNA gene were carried out in separate tubes in an ABI 7700 Prism Sequence Detector (Applied Biosystems) thermocycler. In the light of Figure 1, the quantification of *N*. *caninum* was carried out using interpolation of the Ct (threshold cycle: cycle in which the sample is considered positive of a standard curve constructed from known concentrations of parasite DNA (10⁴-0.1 tachyzoites) against their respective Ct. For the quantification of the host DNA another standard curve was constructed with known genome DNA (500-4 ng). The determination of the Ct value and the number of copies was calculated using the Sequence detector 7700 software (Applied Biosystems).

The sensitivity of the quantitative PCR of our fresh semen samples was 1 tachyzoite per reaction, the contaminated fresh semen being amplified with 10 tachyzoites. The parasite DNA was detected in the cellular fraction.

The described procedure was applied to fresh and frozen semen origination from bulls from artificial insemination centres who were seropositive against *N. caninum.* The parasite DNA was detected in several samples of fresh as well as frozen semen and always in the cellular fraction, where up to 1 tachyzoite in 60 ng of DNA could be quantified.

### Example 4

### Evaluation of the viability of N. caninum in semen samples:

The standardisation of the procedure to determine the sensitivity of the bioassay in semen samples, comprises the evaluation of the infective capacity of *N. caninum* tachyzoites inoculated into fresh semen and semen subjected to a process of dilution in cryopreservatives and freezing.

With the purpose of determining the sensitivity of the bioassay in samples of fresh semen, 250 µl aliquots of bovine semen were contaminated with different quantities of live tachyzoites obtained in cell culture. For this, after counting the viable tachyzoites in a Neubauer chamber with trypan blue at 0.05 %, serial decimal dilutions were made in sterile phosphate buffer (PBS) and different aliquots were contaminated with 1x10⁴, 1x10³, 1x10², 10, 1 and 0 tachyzoites per sample. Immediately afterwards, the seminal fluid and the cellular fraction of the semen of the different aliquots were separated using centrifugation at 1350 x g for 10-15 minutes, the supernatant (seminal fluid) was removed and the sediment was resuspended in 500 µl of PBS with Penicillin G (1000U/ml) and Streptomycin (100 µg/ml). The different aliquots were stored at 4° C for a period less than 30 minutes until the inoculation by the intraperitoneal route into athymic mice of the BALBc *nu*/*nu* strain.

As regards the frozen semen samples, aliquots of fresh semen were mixed with a commercial diluent (a mixture of Tris, glycerol, antibiotics and egg yolk) as a cryopreservative and 250 µl aliquots were prepared, which were contaminated with serial decimal dilutions of live tachyzoites prepared by the same procedure described for fresh semen. Next, the different aliquots were contaminated with 1x10⁵, 1x10⁴, 1x10³, 1x10², 10, 1 and 0 tachyzoites per sample and frozen by immersion in nitrogen vapour for 10 minutes and then they were stored in liquid nitrogen for 48 h. Before their inoculation into mice of the BALBc *nu*/*nu,* strain, the different aliquots were defrosted using direct immersion in a water bath at 37° C and immediately afterwards injected into female mice of the BALBc *nu*/*nu* strain of approximately 20-25 grams in weight. After inoculation the animals were observed daily to detect the appearance of symptoms compatible with neosporosis, including nervous disorders, anorexia and lethargy (Yamage et al., 1996, J. Parasitol. 82: 172-179). When compatible clinical symptoms appeared or 4 months had passed since the inoculation the mice were sacrificed using CO₂ inhalation. In the autopsy the brain was removed in aseptic conditions and DNA was extracted from a portion of approximately 20 mg, using a commercial test: "Genomic-Prep cell and tissue DNA isolation kit" (Amersham Biosciences) following the instructions of the manufacturer and 5 µl (100-200ng) of the resulting DNA solution was used as a pattern for the real time quantitative PCR, as described in Example 3.

The BALBc *nu*/*nu* mice inoculated with the fresh semen contaminated with 1x10² tachyzoites or higher quantities developed symptoms compatible with the infection between 25-30 days post-inoculation. Therefore, the sensitivity of the procedure was 1x10² tachyzoites in samples of fresh semen. Likewise, the mice inoculated with samples of frozen semen contaminated with 1x10⁴ or higher quantities of tachyzoites developed symptoms compatible with the infection at 25-30 days post-inoculation.

## Claims

1. Procedure for the quantification of DNA of the *N. caninum* parasite in bovine semen comprising:
a). extraction of DNA from *N. caninum* present in the bovine semen and
b). quantification by real time PCR with the primers identified as SEQ ID NO 1 and SEQ ID NO 2.

2. Procedure according to claim 1 where the bovine semen may be fresh or frozen.

3. Procedure according to claim 1 where the real time PCR is carried out using the SYBR Green system.

4. Procedure to evaluate the viability of the *N. caninum* parasite in bovine semen samples, analysed according to a previous claim, **characterised by** its carrying out by means of a bioassay in mice of the *nude* or athymic strain, which comprises of the following stages:
a). Separation of the seminal fluid and cellular fraction of the semen,
b). Inoculation of the cellular fraction in mice of the *nude* or athymic strain,
c). Follow up of the inoculated mice for the detection of symptoms compatible with neosporosis and/or subsequent detection-quantification of the DNA of the parasite from the brain by means of using quantitative PCR in real time.

5. Procedure for the evaluation of the viability of the *N. caninum* parasite in samples of bovine semen, according to claims 3 and 4, **characterised in that** the sample is frozen semen with diluent.

6. Procedure for the evaluation of the viability of the *N. caninum* parasite in samples of bovine semen, according to claims 4 and 5, **characterised in that** the animals used in the bioassay are of the BALB/c *nu*/*nu* strain.

## Patentansprüche

1. Verfahren zur Quantifizierung von DNA des Parasiten *N*. *caninum* in Rindersamen, umfassend:
a) Extraktion von DNA aus in dem Rindersamen vorhandenem *N. caninum* und
b) Quantifizierung durch Echtzeit-PCR mit den als SEQ ID NO 1 und SEQ ID NO 2 identifizierten Primern.

2. Verfahren nach Anspruch 1, wobei der Rindersamen frisch oder gefroren sein kann.

3. Verfahren nach Anspruch 1, wobei die Echtzeit-PCR unter Verwendung des SYBR Green-Systems durchgeführt wird.

4. Verfahren zum Bewerten der Lebensfähigkeit des Parasiten *N. caninum* in Rindersamen-Proben, analysiert gemäß einem vorangehenden Anspruch, **dadurch gekennzeichnet, dass** es mittels eines Biotests in Mäusen des Nacktmaus- (*nude*) oder athymischen Stammes durchgeführt wird, welcher die folgenden Stufen umfasst:
a) Trennung der Samenflüssigkeit und der Zellfraktion des Samens,
b) Inokulation der Zellfraktion in Mäuse des Nacktmaus- (nude) oder athymischen Stammes,
c) Nachbetreuung bzw. Überwachung der inokulierten Mäuse zum Nachweis von Symptomen, die mit der Neosporose einhergehen, und/oder anschließende Nachweis-Quantifizierung der DNA des Parasiten aus dem Gehirn unter Verwendung von quantitativer PCR in Echtzeit.

5. Verfahren zur Bewertung der Lebensfähigkeit des Parasiten *N. caninum* in Proben von Rindersamen, nach den Ansprüchen 3 und 4, **dadurch gekennzeichnet, dass** es sich bei der Probe um gefrorenen Samen mit Verdünnungsmittel handelt.

6. Verfahren zur Bewertung der Lebensfähigkeit des Parasiten *N. caninum* in Proben von Rindersamen, nach den Ansprüchen 4 und 5, **dadurch gekennzeichnet, dass** die in dem Biotest verwendeten Tiere zum BALB/c *nu*/*nu*-Stamm gehören.

## Revendications

1. Procédure pour la quantification de l'ADN du parasite de *N. Caninum* dans la semence bovine comprenant:
a) l'extraction de l'ADN du *N. Caninum* présent dans la semence bovine,
b) la quantification par Amplification en chaîne par Polymérase (PCR) en temps réel à l'aide des amorces identifiées SEQ ID N°1 ou SEQ ID N°2.

2. Procédure selon la revendication 1, dans laquelle la semence est fraîche ou congelée.

3. Procédure selon la revendication 1, dans laquelle la PCR en temps réel est mise en oeuvre à l'aide du système SYBR Green.

4. Procédure d'évaluation de la viabilité du parasite de *N. Caninum* dans des échantillons de semence bovine, analysé selon une des revendications précédentes, **caractérisée en ce qu'**elle est mise en oeuvre au moyen d'un test biologique sur des souris de souche nude ou athymique, qui comprend les étapes suivantes:
a) séparation de fluide séminal et de la fraction cellulaire de la semence;
b) inoculation de la fraction cellulaire de souris de souche nude ou athymique;
c) suivi de la souris inoculée en vue de détecter les symptômes compatibles avec la néosporosis et/détection-quantification subséquente de l'ADN du parasite du cerveau à l'aide d'une PCR quantitative en temps réel.

5. Procédure d'évaluation de la viabilité du parasite de *N. Caninum* dans des échantillons de semence bovine, selon les revendications 3 et 4, **caractérisé en ce que** l'échantillon est de la semence congelée avec un diluant.

6. Procédure d'évaluation de la viabilité du parasite de *N. Caninum* dans des échantillons de semence bovine, selon les revendications 3 et 4, **caractérisé en ce que** les animaux utilisés dans le test biologique sont de souche BALB/c nu/nu.
